# EUROPEAN PATENT SPECIFICATION

(11) **EP 1 201 762 B1**
(45) Date of publication and mention of the grant of the patent: **02.03.2005**
(21) Application number: 00949493.1
(22) Date of filing: 21.07.2000
(51) Int. Cl.: C12P 5/02

(54) **PROCESS FOR PRODUCING LYCOPEN**
VERFAHREN ZUR HERSTELLUNG VON LYKOPEN
PROCEDE POUR L'OBTENTION DE LYCOPENE

(30) Priority: 12.08.1999 ES 9901869
(43) Date of publication of application: 02.05.2002
(73) Proprietor: Vitatene, S.A., 24080 Leon (ES)
(72) Inventor: ESTRELLA DE CASTRO, Antonio, E-24080 Leon (ES); COLLADOS DE LA VIEJA, Alfonso J., E-24080 Leon (ES); BERNASCONI, Ermanno, E-24080 Leon (ES); ESTEBAN MORALES, Manuel, E-24080 Leon (ES); GONZALEZ DE PRADO, Emiliano, E-24080 Leon (ES)
(74) Representative: Elzaburu, Alberto de
(86) International application number: PCT/ES2000/000266
(87) International publication number: WO 2001/012832

(56) References cited:
- WO-A1-98/03480
- WO-A1-98/43620

## Description

The present invention relates to a new method of isolating and purifying lycopene, in principle starting from any natural source of biosynthesis and more concretely from submerged cultures of Mucorales fungi such as *Blakeslea, Choanephora* or *Phycomyces* in a crystalline form for human consumption. A method of extraction is described that makes it possible to simplify the recovery process and obtain an increase in purity of the product relative to the methods previously described.

### State of the art

The carotenoid pigments occur widely in nature in fruits and vegetables, giving them their characteristic colour from yellow to dark red in numerous natural substances such as carrot, peppers, tomatoes, flowers or certain microorganisms. They can be divided into two classes: pure hydrocarbons such as the carotenes that include compounds such as β-carotene, alpha-carotene or lycopene, and xanthophylls, which contain an oxygenated function, examples of this type being astaxanthin, capsanthin or cantaxanthin. The two groups of compounds exhibit different behaviour with regard to their physico-chemical properties and different solubility in organic solvents.

All these compounds play an important role in the human diet, and there have been extensive studies of their properties as antioxidants for the prevention of cancer and other human diseases, and as vitamin A precursors. Moreover, on account of their yellow to red coloration, the carotenoids are used as food supplement and colorant in margarine, butter, oils, soups, sauces etc. (Ninet et al. Microbial Technology 2nd Edn. Vol. 1 529-544 (1979) Academic Press NY Eds Peppler HJ and Perlman D.)

Lycopene is a carotenoid that is produced as an intermediate in the biosynthetic pathway of β-carotene and xanthophylls belonging to the group of hydrocarbon carotenoids. Its empirical formula is C₄₀H₅₆, its molecular weight is 536.85 and it has the following molecular formula:

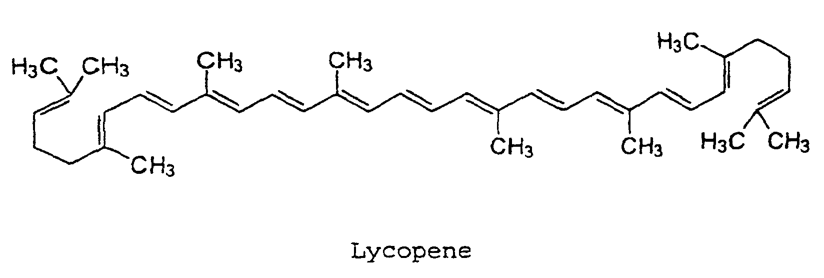

The properties of this compound have been studied extensively. Thus, its antioxidant properties have been described, capturing the free radicals that are produced continuously in the human body, and its application in the prevention of cardiovascular diseases and some types of cancers such as prostate cancer (Giovannucci et al., J Nat. Cancer Inst. 87: 1767-1776 (1995); Stahl et al., Arch Biochem. Biophys 336: 1-9 (1996); Clinton et al., Nutr. Rev. 56: 35-51 (1998)) which has given rise to an increase in demand on the part of consumers and therefore the industry has tried to satisfy this demand by manufacturing lycopene by chemical synthesis.

The production of lycopene as a high-purity compound has been linked in the past to chemical synthesis (US 5208381; US 5166445; US 4105855; US 2842599), processes that are now the subject of debate as it is considered that alternative routes starting from sources of natural origin combined with specific extraction processes prove more advantageous and constitute what is called the "natural route" for obtaining these products.

By the so-called "natural route", lycopene can be obtained starting from plant products such as: tomato, carrot, peppers, vegetable oils, etc. Thus, patent WO 97/48287 describes a method of preparation of lycopene-rich oleoresins from tomatoes by crushing the tomato until a pulp is obtained, extraction of lycopene from the pulp with organic solvents and subsequent elimination of the solvent by evaporation, leading to an oleoresin with a lycopene content in the range 2-10%.

Similar methods of obtaining oleoresins rich in carotenoids in general and lycopene in particular from plants and oils are described in various patents US 5245095 and EP 580745 by precipitation with calcium salts, US 5019668 using a process of transesterification with oils followed by distillation, WO 95/16363 describes fractionation of the tomato in various fractions that include an oleoresin rich in carotenoids, and PCT WO 90/08584 describes the extraction of lycopene using fluids in a supercritical state although the extract obtained is a mixture of various carotenoids and the yields in extraction are very low owing to its low solubility.

In all these cases, owing to the low concentration of lycopene in these natural products and the intracellular distribution of this compound in certain organelles such as chloroplasts or chromoplasts, the extraction yields and product purity obtained are low, as there is production of lycopene-rich oleoresins or dehydrated raw products together with variable amounts of other carotenoid or non-carotenoid compounds. In most cases the methods of extraction described require preparation of the fruit by crushing and extrusion to facilitate solvent extraction and so release the lycopene-rich cellular contents. Finally, in most of the processes described in these patents it is necessary to use organic solvents, which appear as traces in the oleoresin obtained.

Similarly, patent IL 107999 describes the production of oleoresins very rich in lycopene starting from tomato pulp, though just as in the previous cases, lycopene crystals of high purity are not obtained, but lycopene-rich lipid concentrates.

On the other hand, patent WO 97/15554 describes the extraction of carotenoids of plant origin starting from carrots and tomatoes, including lycopene, by the isolation of chloroplasts and chromoplasts, then digestion of the said organelles with hydrolytic enzymes of proteins such as pectins and/or proteases that permit the release of the lycopene joined to various structural proteins. By subsequent alkaline treatment and extraction with alcoholic mixtures of low molecular weight it is possible to obtain lycopene extracts with yield and purity greater than the oleoresins though without obtaining purified crystals of lycopene, but lycopene-rich raw extracts.

In addition, in patent EP 608027 A2, concentrated extracts of lycopene are obtained by the isolation of chromoplasts of tomatoes in which lycopene occurs in crystalline form. These lycopene-rich extracts from tomato chromoplasts are used directly as colorants without subsequent extraction of the lycopene crystals, avoiding change of colour of the lycopene during extraction and making it unnecessary to use organic solvents. According to the method described in that patent, it is not possible to obtain pure lycopene in crystalline form suitable for use in foodstuff or pharmaceutical compositions, but only as food colorant in dehydrated, lyophilized or frozen form.

Another important source of lycopene is certain microalgae that are rich in carotenoids, of the *Dulaniella* type. Various methods of extraction of carotenoids from these organisms and in particular of lycopene have been described, e.g. in US 5378369, US 4713398 and US 4680314, by extraction with organic solvents (chlorocarbonates, hydrocarbons, etc.) or edible oils DE 4342798. A different process is described in PCT WO 98/08584 where a lycopene extract is obtained using CO₂ in the supercritical state, but the extract so obtained is of low purity with respect to lycopene.

Lycopene can be obtained from certain Mucorales fungi such as *Phycomyces, Blakeslea* or *Choanephora* by fermentation in a liquid medium, and compared with the production of lycopene from plant products or algae, these have the advantage of high concentration of this compound, in some cases above 5 wt.% relative to the amount of dry biomass (concentrations higher than those obtained from the best plant varieties), as well as the possibility of biotechnological development of overproducer strains of these microorganisms either by techniques of classical mutagenesis or by application of the new technologies of molecular biology that permit the genetic manipulation of these microorganisms to increase the concentration and production of lycopene as well as elimination of the production of other structurally related carotenoids.

The preparation of crystalline lycopene of high purity from natural sources generally requires, as already mentioned, a stage of extraction with organic solvents or fluids in a supercritical state, and then various additional stages of purification such as chromatography, processes of adsorption and elution and stages of precipitation or crystallization as described for example in patents US 3369974, EP 818255 and EP 242148. In the majority of cases when these stages of subsequent purification are not used and crystallization is effected directly from the extract by evaporation of the solvent until solubility is exceeded, the product obtained is of very low purity and it is subsequently necessary to carry out processes of recrystallization of the lycopene obtained with the problem of low solubility of the product and therefore large quantity of solvent required for effecting the recrystallization stage, as well as considerable losses of yield (NL 6411184, US 4439629)

Patent application WO 96/13178 describes the preparation of concentrates of crystalline lycopene stabilized in a food-compatible liquid medium in which the lycopene is insoluble, such as ethyleneglycol, ethanol or glycerol, obtaining lycopene crystals of small size by grinding, between 1-3 µm, suspended in a liquid medium.

Moreover, patent application WO 98/43620 describes a method of isolation of lycopene crystals from an oleoresin by a process of saponification of various triglycerides and phosphonates at high temperature followed by dilution with water, obtaining lycopene crystals of purity between 75 and 95%. A similar method has been described recently for the preparation of crystals of β-carotene of high purity in international application WO 98/03480 by washing with water, alcohols of low molecular weight or acetone, though its application for the production of lycopene crystals of high purity by a similar method has not been described.

### Summary of the invention

This invention refers to the production of crystalline lycopene of increased purity starting from a natural biosynthesis source, generally and more concretely from a fermentation medium, employing solvents that are regarded as natural. Natural solvents are regarded as those that are, on the one hand, toxicologically harmless and/or those that are included in Class 3 of the ICH (International Conference of Harmonization).

Generally the process comprises the following stages:
1) Separation of the wet biomass from the natural biosynthesis source (fermentation medium, for example).
2) Purification of the wet biomass by treatment with an alcohol with low solubility for lycopene selected among methanol, ethanol, propanol or isopropanol.
3) Separation of the purified biomass from the alcohol.
4) Conditioning of the purified biomass by drying plus its disintegration or breakdown.
5) Solid-liquid extraction of the lycopene with an organic solvent with food or pharmaceutical grade, selected among acyl esters.
6) Concentration of the enriched extract.
7) Precipitation/crystallization by addition of an alcohol with low solubility for lycopene selected among methanol, ethanol, propanol or isopropanol.
8) Filtration.
9) Drying.

The method described here enables us to recover crystalline lycopene with a purity above 90%, preferably above 95% and more preferably above 98%.

### Detailed description of the invention

Owing to the intracellular characteristics of the carotenoid component biosynthesised in fermentation, the recovery process from the culture medium, prepared in accordance with the usual methods, involves separation of the biomass from the medium, with the aim of eliminating of reducing the losses in the medium without biomass.

This separation can be effected by the established methods of A) Filtration, employing the usual technologies with filters, whether band filters, rotary filters, presses etc., in which a barrier constituted by the filter cloth separated the biomass and permits the liquid phase without biomass to pass through, or B) Centrifugation, in which, taking advantage of the density difference between the medium and the biomass (normally greater), a machine is used such as a centrifugal separator, decanter or the like, in which the heavy phase is concentrated and is separated from the liquid phase with the least possible amount of biomass. Reduction of losses and optimization of the characteristics of each respective phase obtaining the largest quantity of biomass with the highest content of dry residue and eliminating most of the fermentation medium, with the smallest amount of active substance, is one of the objectives of this invention.

The resulting wet mycelium contains more than 95% of the carotenoids produced in fermentation, preferably more than 97% and more preferably more than 99%. The content of carotenoids in the aqueous phase is therefore below 5%, preferably below 3% and more preferably below 1%.

This wet solid of the mycelium would be in a position to permit, be means of the subsequent stages, the separation of lycopene, but the finding that, in connection with fermentation, it still has a relatively high percentage of lipophilic components, between 15 and 20% (fatty acids and oils) which cause problems of purification in subsequent stages, leads us to introduce, at this point, a stage of purification of the biomass.

This purification stage involves resuspension of the biomass with a quantity of alcohol: methanol, ethanol, propanol, isopropanol, or any other alcohol in which the solubility of lycopene is very low, in a suitable proportion to give maximum purification of the lipid components. That is, the wet mycelium is resuspended with a quantity of alcohol varying between 1 ml/g and 10 ml/g of wet mycelium. The temperature of resuspension varies between 0°C and the boling point of the alcohol. The contact time varies between 5 minutes and 24 hours. The alcoholic resuspension thus prepared is filtered or centrifuged, so that the content of solids in the filtrate or clarified liquid is practically zero. The resulting wet mycelium, which will contain alcohol and water in various proportions, contains more that 93% of the carotenoids produced in fermentation, preferably more than 95% and more preferably more than 97%.

In the resulting mixture of residues of culture medium with alcohol, the content of carotenoids is less than 2%, and preferably less than 1%. By means of this treatment with alcohol it is possible to eliminate a number of alcohol-soluble lipophilic substances, in variable quantity depending on the characteristics of the culture medium used, carrying out prior purification that is extremely important and that will enable us to obtain a crystalline end product of high purity. Furthermore, the drying process is greatly facilitated by the elimination of a variable proportion of water from the initial net mycelium.

As an alternative to the combination of these two stages of separation of the biomass and its subsequent purification by resuspension, consideration is given to direct mixing of the culture medium with the alcohol in volumetric proportionsbetween 1:1 to 10:1 and maintaining a minimum contact time, achieving a purification effect equivalent to that described for the two-stage alternative, so that the process is simplified by the elimination of an operation of solid-liquid separation.

The fact that the carotenoid product is intracellular means that the purified biomass requires conditioning either by drying plus grinding, drying plus disintegration or direct disintegration of the biomass, which promotes mixing with solvents and facilitates solvent extraction.

The dewatered/purified mycelium is dried. Drying can be effected by the usual batch or continuous processes. The drying temperature varies between room temperature and 150°C, preferably it should not exceed 60°C and more preferably should be below 50°C. The drying time depends on the temperature employed, and varies between 1 hour and 72 hours. Owing to possible decomposition of these carotenoids by oxidation with atmospheric oxygen, it is advisable to carry out this drying operation in absence of oxygen, either under a nitrogen atmosphere or at least in vacuum.

To obtain good accessibility of the solvent to the carotenoid to be extracted, an operation of breakdown of the mycelium is required beforehand, so that the area of contact is maximum. The optimum particle size of the dry, disrupted mycelium must be less than 3 mm, preferably less than 1 mm and more preferably less than 0.5 mm.

Grinding can be effected on the dry product, by means of mechanical systems with rotating or fixed parts: hammers, sieves, etc., by passage through rotating cylinders pressing against one another or by the flash (instantaneous) drying system in a jet mill, where the wet product is fed into a recirculating stream of a gas at high temperature, in such a way that the residence time is the minimum to achieve vaporization of the content of liquid components, and transports the product, owing to the difference in densities, to a cyclone where it is recovered.

During drying and transportation there is also an effect of homogenization as the particles strike the walls.

Various organic solvents can be used for extraction of the lycopene from a conditioned mycelium as described. This invention will refer to the use of food-grade solvents that are regarded as natural, such as acyl esters, preferably ethyl, propyl, isopropyl, butyl and isobutyl acetates, which combine reasonably high solubility for the carotenoid components with compatibility as solvents included in Class 3 of the ICH. These solvents are permissible both at national and at community level, in both the pharmaceutical and the food sectors (RDL12/04/90 and RDL16/10/96).

The extraction temperature varies between room temperature and the boiling point of the solvent, preferably between 40°C and 80°C and more preferably between 60-70°C. The extraction time will be the minimum necessary to achieve solution, between 1 second and 1 hour, preferably between 1 minute and 15 minutes and more preferably less than 10 minutes. The amount of solvent used depends on the temperature and on the richness of the mycelium in carotenoids, and varies between 5 ml/g and 20 ml/g. The number of ex-tractions varies from 1 to 3. The quantity of carotenoids extracted is greater than 85%, preferably greater than 90% and more preferably greater than 95%.

Once the extract rich in carotenoids is obtained, it has to be concentrated to a certain volume. The final concentration of carotenoids in the solvent after concentrating varies preferably between 10 and 40 g/l. The concentration temperature must be below 80°C, preferably below 70°C and more preferably below 50°C. The concentration time must be less than 1 hour, preferably less than 30 min, and more preferably less than 15 min.

Once the extract has been concentrated to the required volume, it is necessary to add an agent that renders the carotenoids insoluble, concretely an alcohol and more concretely methanol, ethanol, isopropyl alcohol, etc., or any other alcohol in which the solubility of lycopene is very low, giving a considerable increase in yield of crystalline lycopene. Addition of alcohol also has a purifying effect, giving a product that is purer than when alcohol is not added. The crystallization time varies between 15 min and 24 hours, preferably between 1 h and 12 h and more preferably between 3 and 8 hours. The crystallization temperature must be below 25°C, and preferably below 5°C.

The crystals can be separated from the mocher liquor by filtration or centrifugation, removing the mother liquor with which the crystals are soaked, and washing with the same alcohol as used for making them insoluble.

The crystals obtained are dried in vacuum at room temperature for at least 1 h until the content of residual solvents meets the specifications for maximum concentration laid down by the legislation mentioned previously and which, in the case of lycopene, is stipulated as a loss on drying < 0.5%.

The purity of the crystals obtained by the method described above corresponds to a content - determined by spectrophotometry by measurement of absorption at 472 nm dissolved in n-hexane (E1% 1cm = 3450) - greater than 95%, with a content of β-carotene less than 3% and a content of other carotenoids less than 2%.

The crystalline product obtained can be handled and marketed as such or forming part of formulations in which the proportion of lycopene could vary between 1 and 85%w/w, preferably 15-50% w/w mixed with various excipients or compounds, as in the case of soya oil, maize oil, olive oil, etc. with various degrees of purity and accompanied by antioxidants of the tocopherol type.

The method of this invention is especially suitable for the recovery of crystalline lycopene from a microbial source, preferably algae, fungi or yeasts, more preferably from fungi of the Mucorales order, and more preferably *B. trispora*.

The exceptional purity achieved in the crystals obtained by the present methodology, and the use of solvents that are regarded as natural, means that these crystals can be used in the food, pharmaceutical or cosmetics industry.

### Example 1

Three litres of fermentation medium, corresponding to a process of biosynthesis in which the biosynthetic pathway is interrupted at the lycopene level, are harvested. The strength of the culture medium is 1.4 g of lycopene per litre. The biomass of this medium is recovered by filtration in a Buchner filter (porcelain funnel supporting a disk of paper or cardboard that acts as the filtering film), obtaining 880 g of wet biomass. The wet biomass is resuspended in 5.2 l of azeotropic isopropanol 85/15 and is stirred for 30 minutes. The purified biomass is returned for recovery by the Buchner filter.
This biomass is dried in a stove under vacuum at temperature below 45°C for 18 hours, until the content of residual solvents is of the order of 1-2%. 150 g of dry and purified biomass is obtained with a lycopene content equivalent to a strength of 2.75%.
The dry biomass is ground in a hammer mill with a 1 mm screen, giving a solid with the same specific strength, and conditioned to permit solvent extraction.
Extraction is carried out by mixing the 150 g of ground biomass with 1500 ml of isobutyl acetate at 70°C, stirring for 5 minutes. The spent biomass is separated from the rich solvent by filtering through a filter plate. The spent biomass is washed with 300 ml of hot isobutyl acetate on the same filter, mixing the two solvents. All of the rich isobutyl acetate is concentrated under vacuum, keeping the temperature below 45°C until the volume is reduced to 200 ml, so that the lycopene has partially crystallized. To complete crystallization and obtain a purer lycopene, 600 ml of isopropanol is added. The mixture is stirred under nitrogen and in the range 0-5°C for 3 hours. It is filtered through a Buchner funnel, washing the crystals with 25 ml of isopropanol on the Buchner. The crystals are collected and dried, obtaining 3.3 g of lycopene crystals with a purity of 94%.

### Example 2

A quantity of fermentation medium of the order of 500 litres is cultivated. It is mixed directly with 500 l of azeotropic isopropanol with water 85-15. After stirring for 30 min, the biomass is separated from the liquid by filtering on a filter press. About 130 kg of wet, purified biomass is collected.

This biomass is dried in a stove under vacuum until the content of residual solvents is of the order of 1-2%. Temperature below 45°C and time of the order of 12-24 h. 30 kg of biomass is obtained with a lycopene content equivalent to a specific strength of 3%, with purity practically the same as in Example 1, though slightly lower.

The dry biomass is ground in a hammer mill with a 1 mm screen, obtaining a solid with the same specific strength, and conditioned to permit solvent extraction. Extraction is carried out by mixing the 30 kg of ground solid with 350 l of isobutyl acetate at 70°C, stirring for 15 min. The spent biomass is separated from the rich solvent by filtering on a filter plate. The spent biomass is washed with 100 l of hot isobutyl acetate on the same filter. The filtrate is mixed with the previous solvent. All of the rich isobutyl acetate is concentrated under vacuum, keeping the temperature below 45°C, until the volume is reduced to 65 l, so that the lycopene has partially crystallized. To complete crystallization of the lycopene, 180 l of isopropanol is added. The mixture is stirred for 3 h while it is cooled to 0-5°C. It is filtered on a Buchner, and the lycopene crystals are collected and dried. 500 g of product is obtained with a purity of 94% according to spectrometry.

## Claims

1. Method of obtaining lycopene from any natural source of biosynthesis, **characterized in that** is comprises sequentially the following stages:
• Treatment of the natural source of biosynthesis directly with an alcohol with low solubility for lycopene selected among methanol, ethanol, propanol or isopropanol and separation of a wet, purified biomass.
• Conditioning of the wet, purified biomass by drying plus disintegration or disruption.
• Solid-liquid extraction of the lycopene contained in the purified biomass with an organic solvent with food or pharmaceutical grade, selected among acyl esters.
• Concentration of the enriched lycopene extract.
• Precipitation/crystallization of the lycopene from the concentrated extract by addition of an alcohol with low solubility for lycopene selected among methanol, ethanol, propanol or isopropanol.
• Filtration.
• Drying.

2. Method according to claim 1, **characterized in that** the natural source of biosynthesis is a microbial fermentation medium.

3. Method according to any of the claims 1 and 2, **characterized in that** the biomass is purified at alcohol:biomass ratio (v/v) of the order of 1:1 to 10:1.

4. Method according to any of the claims 1 to 3, **characterized in that** the alcohol is used at a temperature between 0°C and that corresponding to its boiling point, preferably between 10 and 50°C and more preferably between room temperature and 40°C.

5. Method according to claim 1, **characterized in that** a solvent of the ester type, preferably ethyl acetate, propyl acetate, isopropyl acetate, butyl acetate or isobutyl acetate, is used in the stage of solid-liquid extraction.

6. Method according to claim 5, **characterized in that** a quantity of solvent of the order of 5 to 20 volumes by weight of biomass, preferably 10 to 15 volumes by weight, is used.

7. Method according to either of the claims 5 or 6, **characterized in that** the solvent is used at temperatures ranging between room temperature and its boiling point, preferably between 40 and 80°C and more preferably between 60 and 70°C.

8. Method according to any of the claims 5 to 7, **characterized in that** the solvent is used in such a way that the time of contact with the lycopene ranges between 1 second and 1 hour, preferably 1-15 minutes and more preferably is less than 10 min.

9. Method according to claim 1, **characterized in that** precipitation of the concentrated product after extraction is brought about by adding a compound in which the lycopene has low solubility, such as methanol, ethanol, or isopropanol, and in which the substances of lipophilic character that accompany the lycopene remain dissolved.

10. Method as in claim 1, according to which the product obtained can be handled and marketed as such in crystalline form or forming part of formulations in which the proportion of lycopene varies between 1 and 85%, preferably in proportions of the order of 15 to 50% w/w, optionally mixed with various excipients or compounds, as in the case of soya oil, maize oil and olive oil, of various degrees of acidity, and preferably virgin or extra virgin olive oil, with optional addition of antioxidants of the tocopherol type.

## Patentansprüche

1. Verfahren zum Erhalt von Lycopen von einer natürlichen Biosynthesequelle, **dadurch gekennzeichnet, dass** es im Wesentlichen die folgenden Stufen umfasst:
- Direkte Behandlung der natürlichen Biosynthesequelle mit einem Alkohol mit geringer Löslichkeit für Lycopen, ausgewählt aus Methanol, Ethanol, Propanol oder Isopropanol, und Abtrennen einer nassen, gereinigten Biomasse.
- Konditionieren der nassen gereinigten Biomasse durch Trocknen plus Aufschluss oder Unterbrechung.
- Fest-Flüssig-Extraktion des in der gereinigten Biomasse enthaltenen Lycopens mit einem arzneimittel- oder nahrungsmittelgeeigneten organischen Lösungsmittel, ausgewählt aus Acylestem.
- Konzentrieren des angereicherten Lycopenextrakts.
- Ausfällung(Kristallisation des Lycopens aus dem konzentrierten Extrakts durch Zugabe eines Alkohols mit geringer Löslichkeit für Lycopen, ausgewählt aus Methanol, Ethanol, Propanol oder Isopropanol.
- Filtration.
- Trocknen.

2. Verfahren nach Anspruch 1, **dadurch gekennzeichnet, dass** die natürliche Biosynthesequelle ein Mikrobenfermentationsmedium ist.

3. Verfahren nach einem der Ansprüche 1 und 2, **dadurch gekennzeichnet, dass** die Biomasse mit einem Verhältnis von Alkohol:Biomasse (V/V) in der Ordnung 1:1 bis 1:10 gereinigt wird.

4. Verfahren nach einem der Ansprüche 1 bis 3, **dadurch gekennzeichnet, dass** der Alkohol bei einer Temperatur zwischen 0°C und derjenigen, die seinem Siedepunkt entspricht, vorzugsweise zwischen 10 und 50°C und stärker bevorzugt zwischen Raumtemperatur und 40°C verwendet wird.

5. Verfahren nach Anspruch 1, **dadurch gekennzeichnet, dass** ein Lösungsmittel des Ester-Typs, vorzugsweise Ethylacetat, Propylacetat, Isopropylacetat, Butylacetat oder Isobutylacetat in der Stufe der Fest-Flüssig-Extraktion verwendet wird.

6. Verfahren nach Anspruch 5, **dadurch gekennzeichnet, dass** eine Lösungsmittelmenge in der Ordnung von 5 bis 20 Volumina, vorzugsweise 10 bis 15 Volumina, bezogen auf das Gewicht der Biomasse, verwendet wird.

7. Verfahren nach entweder Anspruch 5 oder 6, **dadurch gekennzeichnet, dass** das Lösungsmittel bei Temperaturen im Bereich zwischen Raumtemperatur und seinem Siedepunkt, vorzugsweise zwischen 40 und 80°C und stärker bevorzugt zwischen 60 und 70°C verwendet wird.

8. Verfahren nach einem der Ansprüche, **dadurch gekennzeichnet dass** das Lösungsmittel in solcher Weise verwendet wird, dass die Kontaktzeit mit dem Lycopen im Bereich zwischen 1 Sekunde und 1 Stunde, vorzugsweise 1-15 Minuten liegt und stärker bevorzugt weniger als 10 Min. beträgt.

9. Verfahren nach Anspruch 1, **dadurch gekennzeichnet, dass** die Ausfällung des konzentrierten Produkts nach der Extraktion durch Zugabe einer Verbindung, in welcher das Lycopen eine geringe Löslichkeit aufweist, wie Methanol, Ethanol oder Isopropanol, und in welcher die das Lycopen begleitenden Substanzen lipophilen Charakters gelöst bleiben, bewirkt wird.

10. Verfahren nach Anspruch 1, gemäß welchem das erhaltene Produkt als solches in kristalliner Form oder als Formteil von Formulierungen, in welchen der Lycopenanteil zwischen 1 und 85%, vorzugsweise in Anteilen in der Ordnung von 15 bis 50% G/G variiert, gegebenenfalls gemischt mit verschiedenen Exzipienten oder Verbindungen, wie im Falle von Sojaöl, Maisöl und Olivenöl mit verschiedenen Säuregraden und vorzugsweise natives Olivenöl oder natives Olivenöl extra mit optionaler Zugabe von Antioxidationsmitteln des Tocopherol-Typs gehandhabt und vertrieben werden kann.

## Revendications

1. Procédé d'obtention de lycopène à partir de toute source naturelle de biosynthèse, **caractérisé en ce qu'**il comprend les étapes successives suivantes :
- le traitement de la source naturelle de biosynthèse directement avec un alcool dans lequel le lycopène est faiblement soluble, choisi parmi le méthanol, l'éthanol, le propanol et l'isopropanol, et la séparation d'une biomasse humide, purifiée,
- la mise de la biomasse humide, purifiée, sous une forme appropriée par séchage et désintégration ou division,
- l'extraction solide-liquide du lycopène contenu dans la biomasse purifiée, à l'aide d'un solvant organique de qualité alimentaire ou pharmaceutique, choisi parmi les esters acylés,
- la concentration de l'extrait enrichi en lycopène,
- la précipitation/cristallisation du lycopène à partir de l'extrait concentré par addition d'un alcool dans lequel le lycopène est faiblement soluble, choisi parmi le méthanol, l'éthanol, le propanol et l'isopropanol,
- la filtration,
- le séchage.

2. Procédé selon la revendication 1, **caractérisé en ce que** la source naturelle de biosynthèse est un milieu de fermentation microbienne.

3. Procédé selon la revendication 1 ou 2, **caractérisé en ce qu'**on réalise la purification de la biomasse à un rapport alcool :biomasse (v/v) de l'ordre de 1 : 1 à 10 :1.

4. Procédé selon l'une quelconque des revendications 1 à 3, **caractérisé en ce qu'**on utilise l'alcool à une température comprise entre 0°C et son point d'ébullition, de préférence entre 10 et 50°C et encore mieux entre la température de la salle et 40°C.

5. Procédé selon la revendication 1, **caractérisé en ce qu'**on utilise dans l'étape d'extraction solide-liquide, un solvant du type ester, de préférence l'acétate d'éthyle, l'acétate de propyle, l'acétate d'isopropyle, l'acétate de butyle ou l'acétate d'isobutyle.

6. Procédé selon la revendication 5, **caractérisé en ce qu'**on utilise une quantité de solvant de l'ordre de 5 à 20 volumes par unité de poids de biomasse, de préférence de 10 à 15 volumes par unité de poids de biomasse.

7. Procédé selon la revendication 5 ou 6, **caractérisé en ce qu'**on utilise le solvant à une température comprise entre la température de la salle et son point d'ébullition, de préférence entre 40 et 80°C et encore mieux entre 60 et 70°C.

8. Procédé selon l'une quelconque des revendications 5 à 7, **caractérisé en ce qu'**on utilise le solvant de telle manière que la durée de contact avec le lycopène est comprise entre 1 seconde et 1 heure, de préférence entre 1 et 15 minutes, et est de préférence inférieure à 10 minutes.

9. Procédé selon la revendication 1, **caractérisé en ce qu'**on réalise la précipitation du produit concentré après extraction, par addition d'un composé dans lequel le lycopène est faiblement soluble, tel que le méthanol, l'éthanol ou l'isopropanol, et dans lequel les substances à caractère lipophile qui accompagnent le lycopène restent dissoutes.

10. Procédé selon la revendication 1, selon lequel le produit obtenu peut être manipulé et commercialisé tel quel sous forme cristalline, ou faire partie de formulations dans lesquelles la proportion de lycopène est de 1 à 85% p/p, de préférence de 15 à 50% p/p, éventuellement en mélange avec divers excipients ou composés, tels que de l'huile de soja, de l'huile de maïs et de l'huile d'olive, de divers degrés de pureté, et de préférence de l'huile d'olive vierge ou extra vierge, des antioxydants du type tocophérol étant éventuellement ajoutés.
